(19) Europäisches Patentamt
European Patent Office
Office européen des brevets

(11) **EP 0 830 876 B1**

(12) **EUROPÄISCHE PATENTSCHRIFT**

(45) Veröffentlichungstag und Bekanntmachung des Hinweises auf die Patenterteilung:
**26.03.2003 Patentblatt 2003/13**

(51) Int Cl.7: **A61N 1/37**, A61N 1/368

(21) Anmeldenummer: **97114964.6**

(22) Anmeldetag: **29.08.1997**

(54) **Implantierbare Vorrichtung zur Tachykardie-Früherkennung und -Unterdrückung beim Herzen**

Implantable device for early detection and suppression of a cardiac tachycardia

Dispositif implantable pour la détection précoce et suppression d'une tachycardie cardiaque

(84) Benannte Vertragsstaaten:
**CH DE FR LI NL**

(30) Priorität: **20.09.1996 DE 19638581**

(43) Veröffentlichungstag der Anmeldung:
**25.03.1998 Patentblatt 1998/13**

(73) Patentinhaber: **BIOTRONIK Mess- und Therapiegeräte GmbH & Co Ingenieurbüro Berlin**
**12359 Berlin (DE)**

(72) Erfinder:
• **Bolz, Armin, Dr.**
**91054 Buckenhof (DE)**
• **Bartels, Klaus, Dr.**
**10115 Berlin (DE)**

• **Flach, Erhard**
**12305 Berlin (DE)**
• **Hintsche, Rainer, Dr.**
**10119 Berlin (DE)**
• **Paeschke, Manfred**
**16352 Basdorf (DE)**

(74) Vertreter: **Hübner, Gerd, Dipl.-Phys. et al Rau, Schneck & Hübner Patentanwälte Königstrasse 2 90402 Nürnberg (DE)**

(56) Entgegenhaltungen:
EP-A- 0 538 990    US-A- 4 088 140
US-A- 4 280 502    US-A- 4 354 497
US-A- 5 267 560    US-A- 5 476 503

**Beschreibung**

Die Erfindung betrifft eine implantierbare Vorrichtung zur Tachykardie-Früherkennung und -Unterdrückung beim Herzen.

Zum Hintergrund der vorliegenden Erfindung ist kurz auf die als Tachykardie bezeichnete Herzrhythmusstörung einzugehen, bei der die Herzrate über ein physiologisch sinnvolles Maß angestiegen ist. Dabei erfolgen die einzelnen Kontraktionen des Herzens so schnell, daß pro Kontraktion keine ausreichende Blutmenge befördert wird. Die Herzratenerhöhung wird also durch das Absinken des Schlagvolumens überkompensiert.

Grundsätzlich sind ventrikuläre Tachykardie-Zustände gefährlich, da die Pumpleistung des Herzens hauptsächlich vom Ventrikel erbracht wird, während das Atrium nur in sehr begrenztem Maße zur Blutförderung beiträgt.

Ventrikuläre Tachykardie-Zustände können in unterschiedliche Schweregrade eingeteilt werden. Eine erste Stufe ist durch Veränderungen in der Morphologie der elektrischen Reizsignale der Herzmuskelzellen und Unregelmäßigkeit im leicht erhöhten Herzrhythmus gekennzeichnet.

Eine zweite Stufe ist das sogenannte Herzflattern mit einer noch nicht lebensbedrohlich angestiegenen Herzrate. Die Abnahme der Pumpleistung kann dabei jedoch bereits zu einer Ohnmacht des Patienten führen.

Die schwerste Stufe ist das sogenannte Herzflimmern, bei dem die drastische Abnahme der Pumpleistung innerhalb kürzester Zeit zum Tod des Patienten führt.

Bis heute wurden zur Therapie bei Tachykardie-Zuständen verschiedene, an den Schweregrad der Tachykardie angepaßte Maßnahmen ergriffen. So kann in einem weniger bedrohlichen Stadium das Einsetzen einer Tachykardie häufig durch sogenannte antitachykarde Stimulation mit für Herzschrittmacher üblichen Stimulationsamplituden von einigen Volt unterdrückt werden. Das stabil, jedoch zu schnell schlagende Herz wird dadurch wieder "eingefangen", wozu Stimulationsimpulse in bestimmten Zeitabfolgen eingesetzt werden. Voraussetzung für den Erfolg einer solchen gemäßigten Therapie ist die rechtzeitige Erkennung und Einstufung eines solchen Tachykardie-Zustandes.

Im Falle von Herzflimmern (Fibrillation) jedoch wirkt in der Regel nur ein Defibrillationsschock von einigen Hundert Volt, falls diese Spannung direkt im Herzen angelegt werden kann. Defibrillationsschocks, die außerhalb des Körpers - beispielsweise über Brustelektroden - angelegt werden, müssen sogar Amplituden von einigen kV aufweisen.

Ein Defibrillationsschock führt im Erfolgsfall zu einer elektrischen Erregung aller Herzmuskelzellen, so daß sich anschließend alle Zellen gleichzeitig in der sogenannten Refraktärperiode befinden. Während dieser als "Totzeit" zu bezeichnenden Refraktärperiode wird jegliche Reizleitung innerhalb des Herzens unterbrochen, also auch diejenige, die zuvor zur Fibrillation geführt hat. Letztere kann z.B. auf einer kreisenden Selbsterregung beruhen , bei der eine Erregungsfront zyklisch um eine krankhaft veränderte Herzgewebestelle - beispielsweise um eine Infarktnarbe - herumläuft.

Zusammenfassend stellt der Defibrillationsschock also eine Art "Reset" des ganzen Herzmuskelapparates dar, der sehr drastisch und schmerzhaft für den Patienten ist. Es ist daher anzustreben, daß Tachykardie-Zustände möglichst früh erkannt und Gegenmaßnahmen ergriffen werden können, um das Eintreten von Fibrillationen zu unterbinden.

Für diese Früherkennung ist die Detektion hoher Herzraten allein nicht ausreichend, da insbesondere im Bereich niedriger Herzraten tachykarde Zustände nicht von natürlichen Steigerungen der Herzrate aufgrund physischer Belastung unterschieden werden können. Deshalb werden zur Früherkennung von Tachykardien im Stand der Technik vor allem zwei Zielrichtungen verfolgt. Es ist dies zum einen die auf einem Herzschrittmacher implementierte Auswertung der sogenannten Herzratenvariabilität in EKG-Diagrammen. Dabei sollen Unregelmäßigkeiten oder bestimmte Beschleunigungsmuster des Herzrhythmus erkannt werden, die regelmäßig bei Beginn des eigentlichen Tachykardie-Zustandes auftreten. Nachteilig bei diesem Verfahren sind die verhältnismäßig hohen Anforderungen an Rechenund Speicherkapazität, wie sie durch heutige Implantate nicht ausreichend erfüllt werden.

Zum anderen wird zur Tachykardie-Früherkennung eine Auswertung der Aktionspotentiale von Herzmuskelzellen herangezogen. Deren Morphologie unterliegt bereits frühzeitig vor Beginn einer Tachykardie bestimmten Veränderungen. Die Implementation solcher Auswerteverfahren ist mangels ausreichender Erkenntnisse jedoch noch nicht in die Praxis umgesetzt, wobei im übrigen ebenfalls wieder zu befürchten ist, daß die Anforderungen an Rechen- und Speicherkapazitäten nach den heutigen Maßstäben zu hoch sind, als daß das entsprechende Verfahren auf einem Implantat implementiert werden könnte.

Dokument US-A-5 476 503 offenbart eine Vorrichtung gemäß Präambel des Anspruchs 1.

Ausgehend von der geschilderten Problematik liegt der Erfindung die Aufgabe zugrunde, eine implantierbare Vorrichtung bereitzustellen, mit deren Hilfe eine zuverlässige Tachykardie-Früherkennung und -Unterdrückung möglich ist.

Zur Lösung dieser Aufgabe ist eine implantierbare Vorrichtung vorgesehen, die versehen ist mit

-    einer in Kontakt mit dem Herzmuskelgewebe stehenden Mikroelektroden-Anordnung zur Erfassung von Reizleitungspotentialen im Herzmuskelgewebe,
-    einer mit der Mikroelektroden-Anordnung in Verbindung stehenden Meßeinrichtung zur Bestimmung der Refraktärzeit der Herzmuskelzellen im überwachten Herzbereich,

- einer mit der Mikroelektroden-Anordnung in Verbindung stehenden Meßeinrichtung zur Bestimmung der Reizleitungsgeschwindigkeit im überwachten Herzmuskelbereich,

- einer Recheneinrichtung zur Bestimmung des Produktwertes aus Refraktärzeit und Reizleitungsgeschwindigkeit,

- einem Komparator zum Vergleich des Produktwertes mit einem Tachykardie-Grenzwert, dessen Unterschreiten einen Tachykardie-gefährdeten Zustand des Herzens signalisiert,

- einer Stimulationsvorrichtung zur Erzeugung einer antitachykarden Stimulation bei Erfassung eines Tachykardie-gefährdeten Zustandes insbesondere über die Mikroelektroden-Anordnung, und

- einer Steuereinrichtung zur Steuerung der vorrichtungsinternen Meß- und Auswerteabläufe.

Die Erfindung geht von einem anderen Ansatz zur Früherkennung von Tachykardien aus. Untersuchungen haben nämlich gezeigt, daß das Verhältnis der Reizleitungsgeschwindigkeit innerhalb des Herzmuskelgewebes zur Refraktärzeit der Herzmuskelzellen als kritische Größe zur Tachykardie-Früherkennuung heranzuziehen ist. Es wurde festgestellt, daß tachykardiekritische Herzzustände dadurch charakterisiert sind, daß der Wert des Produktes der Reizleitungsgeschwindigkeit und der Refraktärzeit unterhalb eines bestimmten Schwellwertes liegt, der im folgenden als Tachykardie-Schwellwert bezeichnet werden soll. Dies läßt sich diagrammatisch erläutern, wobei auf das Ausführungsbeispiel verwiesen wird.

Wird nun also erfindungsgemäß die Refraktärzeit und die Reizleitungsgeschwindigkeit im überwachten Herzmuskelgewebe durch eine Mikroelektroden-Anordnung erfaßt, genügt ein Vergleich des durch die Recheneinrichtung bestimmten Produktwertes dieser beiden Größen mit dem Tachykardie-Schwellwert, um entscheiden zu können, ob das Herz sich in einem Tachykardie-gefährdeten Zustand befindet. Dies kann von der implantierten Vorrichtung automatisch durchgeführt werden, wonach im Bedarfsfall über eine Stimulationsvorrichtung antitachykarde Stimulationen erzeugt werden können.

Ebenfalls anhand von Grenzwerten kann die Einstufung eines Tachykardie-gefährdeten Zustandes nach seiner Bedrohlichkeit für den Patienten automatisch vorgenommen und daraus eine geeignete Therapie ausgewählt werden.

Bevorzugte Ausführungsformen, weitere Merkmale, Einzelheiten und Vorteile der Erfindung sind den Unteransprüchen und der nachfolgenden Beschreibung entnehmbar, in der ein Ausführungsbeispiel des Erfindungsgegenstandes anhand der beigefügten Zeichnungen näher erläutert wird. Es zeigen:

Fig. 1 ein Kurvendiagramm zur Darstellung des Zusammenhanges zwischen Reizleitungsgeschwindigkeit und Refraktärzeit bei der erfindungsgemäßen Tachykardie-Früherkennung,

Fig. 2 ein Zeitdiagramm des monophasischen Aktionspotentials von Herzmuskelzellen,

Fig. 3 ein Blockdiagramm einer erfindungsgemäßen Vorrichtung zur Tachykardie-Früherkennung und -Unterdrückung,

Fig. 4 eine Draufsicht auf eine Mikroelektroden-Anordnung der Vorrichtung gemäß Fig. 3, und

Fig. 5 eine vergrößerte perspektivische Darstellung einzelner Elektrodenarme der Mikroelektroden-Anordnung gemäß Fig. 4.

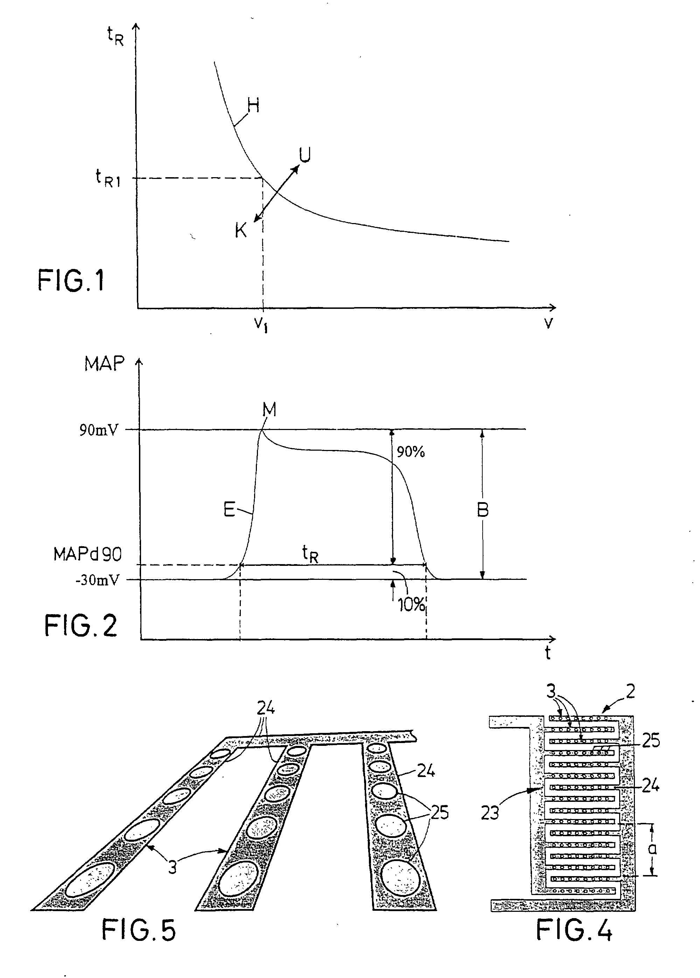

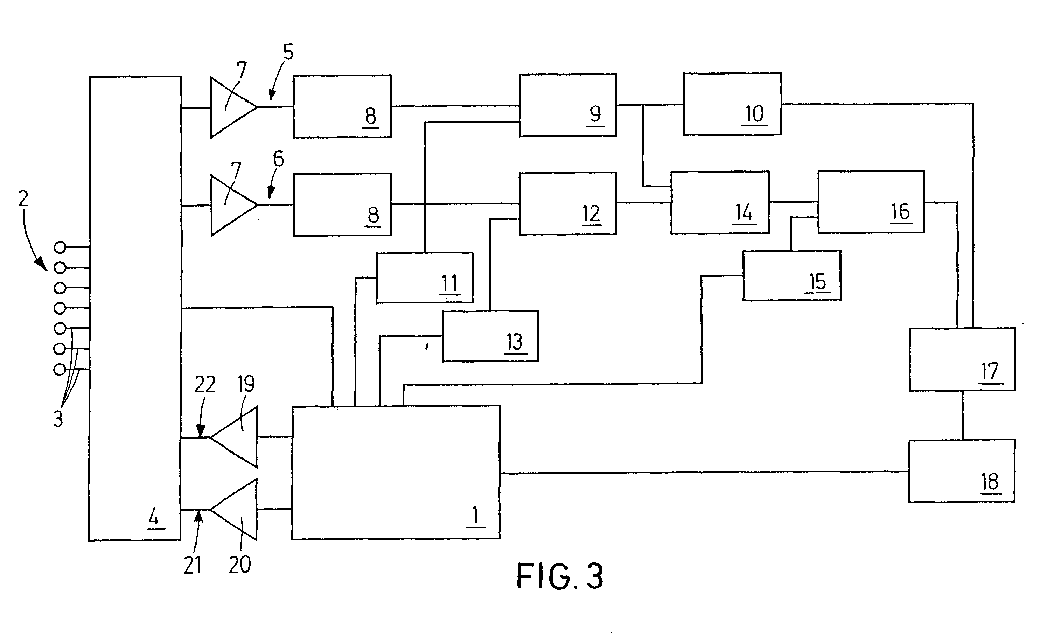

In dem Diagramm gemäß Fig. 1 sind auf der Ordinate die Reizleitungsgeschwindigkeit v im überwachten Herzmuskelgewebe und auf der Abszisse die Refraktärzeit $t_R$ qualitativ aufgetragen. Die in das Diagramm eingetragene Hyperbel H stellt bildlich den Tachykardie-Schwellwert T dar, wobei für kritische Herzzustände gilt:

$$v \times t_R < T$$

Bei kritischen Herzzuständen liegt also das Produkt der beiden Größen v und $t_R$ in dem Gebiet K unterhalb der Hyperbel H. Für unkritische Zustände liegt das Produkt im Bereich U oberhalb der Hyperbel H. Praktisch umgesetzt bedeutet diese Aussage, daß für einen bestimmten Wert $t_{R1}$ der Refraktärzeit die Reizleitungsgeschwindigkeit größer als ein Grenzwert $v_1$ sein muß. In diesem Falle ist die Reizleitung so schnell, daß im Falle einer umlaufenden, "kreisenden" Erregungsfront E diese die Ausgangszelle noch während der Refraktärzeit erreicht. Damit kann keine selbsterregte Stimulation stattfinden, die zu einem Tachykardiezustand führen würde. Ist die Reizleitungsgeschwindigkeit v kleiner als der kritische Wert $v_1$ so ist die Erregungsfront E so langsam, daß sie besagte Ausgangszelle erst nach Ablauf der Refraktärzeit erreicht, womit die Zelle wiederum stimuliert wird und sich ein Tachykardiezustand einstellen kann.

Anhand von Fig. 2 ist die Definition der Refraktärzeit $t_R$ zu erläutern. Dieses Diagramm stellt das sogenannte monophasische Aktionspotential MAP einer Herzmuskelzelle aufgetragen gegenüber der Zeit t dar. Das MAP zeigt die Erregungsfront E der Zelle in Form der steil ansteigenden Flanke. Nach dem Potentialmaximum M von typischerweise 90 mV klingt das MAP mit der gezeigten Kurvenform auf das Ruhepotential von typisch - 30 mV ab.

Wie aus Fig. 2 hervorgeht, ist die Refraktärzeit

$t_R$ als die Periode definiert, innerhalb derer der Wert des MAP's größer als 10% der absoluten MAP-Bandbreite B (Fig. 2) ist (sog. "MAPd90-Wert").

Die Messung der Reizleitungsgeschwindigkeit v und der Refraktärzeit $t_R$ wird mittels eines einzigen Mikroelektroden-Gatters 23 durchgeführt, wie dies anhand von Fig. 4 und 5 noch näher erläutert wird. Unter Bezugnahme auf Fig. 3 soll davor der Aufbau der erfindungsgemäßen Vorrichtung zur Tachykardie-Früherkennung erläutert werden.

Gemäß der funktionsschematischen Darstellung der Vorrichtung nach Fig. 3 weist diese als Herzstück eine Steuereinrichtung 1 auf, die in üblicher Weise auf Mikroprozessorbasis realisiert ist und mit entsprechenden Ablaufprogrammen die vorrichtungsinternen Meß- und Auswerteabläufe steuert, wie sie noch näher erläutert werden.

Zur Erfassung der Reizleitungspotentiale im Herzmuskelgewebe ist nun eine damit in Kontakt stehende Mikroelektroden-Anordnung 2 vorgesehen, die in Fig. 3 schematisch angedeutete bzw. in Fig. 4 und 5 dargestellte Elektrodenarme 3 aufweist. Die Mikroelektroden-Anordnung 2 kann (wie nicht dargestellt ist) auf einer in das Ventrikel oder Atrium der rechten Herzseite einführbaren Elektrodensonde positioniert sein, wie dies bei Elektrodensonden von Herzschrittmachern oder Defibrillatoren bekannt ist.

Zur Selektierung eines zur Messung aktivierten Paars von Elektrodenarmen 3 dient eine Beschaltungseinheit 4, die von der Steuereinrichtung 1 gesteuert wird. Damit können zwei bestimmte Elektrodenarme 3 der Mikroelektroden-Anordnung 2 auf die beiden Eingangskanäle 5, 6 der Vorrichtung geschaltet werden. In beiden Eingangskanälen 5, 6 sind jeweils Eingangsverstärker 7 und Bandpaßfilter 8 zur Signalaufbereitung eingeschaltet. Dem Eingangskanal 5 ist ein erster Schwellwert-Komparator 9 und diesem wiederum ein Refraktärzeit-Glied 10 zugeordnet. Eingangskanal 5 mit Schwellwert-Komparator 9 und Refraktärzeit-Glied 10 bilden praktisch eine Meßeinrichtung zur Bestimmung der Refraktärzeit der Herzmuskelzellen. Dazu wird dem Schwellwert-Komparator 9 von der Steuereinrichtung 1 über eine Schwellwertvorgabe 11 der Schwellwert MAPd90 vorgegeben und mit dem von der Mikroelektroden-Anordnung 2 ermittelten MAP verglichen. Das Refraktärzeit-Glied 10 bestimmt dabei die Periode, in der der MAP-Wert größer als der MAPd90-Wert ist, wie dies in Fig. 2 dargestellt wurde. Die Periodendauer entspricht definitionsgemäß der Refraktärzeit $t_R$.

Dem zweiten Eingangskanal 6 ist ein zweiter Schwellwert-Komparator 12 zugeordnet, dem über die Schwellwert-Vorgabe 13 wiederum der MAPd90-Wert als Schwellwert für die Berechnung der Reizleitungszeit vorgegeben wird. Die beiden Schwellwert-Komparatoren 9 und 12 sind mit einem weiteren Zeitglied, nämlich dem Reizleitungszeit-Glied 14 verbunden, das die Zeitspanne bestimmt, die zwischen dem Zeitpunkt des Erreichens der MAPd90-Schwelle im ersten und zweiten Schwellwert-Komparator 9 und 12 vergeht. Diese Zeitspanne entspricht der Zeitdauer, die die Erregungsfront E vom einen selektierten Elektrodenarm 3 zum zweiten selektierten Elektrodenarm 3 benötigt. Über die Steuereinrichtung 1 ist wiederum aus einem (nicht dargestellten) Speicher die Entfernung a (Fig. 4) zwischen diesen beiden Elektrodenarmen 3 abrufbar, so daß durch diese Entfernungsvorgabe 15 und der vom Reizleitungszeit-Glied 14 ermittelten Reizleitungszeit in einem Rechenglied 16 die Reizleitungsgeschwindigkeit als Quotient aus diesen Größen ermittelt werden kann. Der zweite Eingangskanal 6 mit dem Schwellwert-Komparator 9, dem Reizleitungszeit-Glied 14, der Entfernungsvorgabe 15 und dem Rechenglied 16 dient praktisch als Meßeinrichtung zur Bestimmung der Reizleitungsgeschwindigkeit im überwachten Herzmuskelbereich.

Die vom Refraktärzeit-Glied 10 und dem Rechenglied 16 ermittelten Werte für die Reizleitungsgeschwindigkeit v im überwachten Herzmuskelbereich und die Refraktärzeit der dortigen Herzmuskelzellen werden einer Recheneinrichtung 17 zugeführt, die daraus den Produktwert bestimmt. In dem dritten Schwellwert-Komparator 18 wird verglichen, ob dieser Produktwert größer oder kleiner einem Tachykardie-Schwellwert ist, es wird also festgestellt, ob sich das Herz in einem tachykardiegefährdeten Zustand (Produktwert im Bereich K unter der Hyperbel gemäß Fig. 1) oder in einem unkritischen Zustand (Bereich U oberhalb der Hyperbel H) befindet.

Wird ein tachykardiegefährdeter Zustand festgestellt, wird von der Steuereinrichtung 1 über die beiden jeweils einen Ausgangsverstärker 19, 20 aufweisenden Ausgangskanäle 21, 22 eine antitachykarde Stimulation über die Mikroelektroden-Anordnung 2 dem Herzen aufgegeben.

Anhand von Fig. 4 und 5 wird kurz der Aufbau der Mikroelektroden-Anordnung 2 beschrieben. Die Mikroelektroden-Anordnung 2 ist als planares Mikroelektroden-Gatter 23 (ein sogenanntes interdigitales Array (IDA)) ausgestaltet, das kammartig ineinandergreifende, fingerartige Elektrodenarme 3 aufweist. Jeder Elektrodenarm 3 besteht aus einem leitenden Streifen aus Edelmetall (z.B. Platin, Iridium oder dergleichen), die in eigens präparierte Oberflächen auf Siliciumchips eingelegt und mit einer biokompatiblen, fluidresistenten, elektrisch isolierenden Schicht 24 abgedeckt sind. Auf den Fingerstrukturen weist die in Draufsicht kammartige Schichtkonfiguration Öffnungen 25 mit einem Durchmesser von 0,5 bis 10 μm auf, die einen elektrischen Kontakt zum Zellgewebe ermöglichen. Diese Öffnungen stellen die eigentlichen Mikroelektroden dar. Das gesamte Gatter weist eine Größenordnung von etwa 1 x 1 mm$^2$ auf.

Die fingerartige Struktur gestattet sowohl die Abnahme von orts- und zeitabhängigen Potentialen hoher Auflösung als auch die Abgabe von Strompulsen hoher Stromdichte an das Zellgewebe, was für jedes Fingersystem aufgrund der von der Steuereinrichtung 1 gesteuerten Beschaltungseinheit 4 individuell oder simultan geschehen kann.

Vorteilhaft ist dabei die gleichzeitig mögliche Stimulation und Messung an verschiedenen Elektrodenarmen 3 des Mikroelektroden-Gatters 23. Die Elektrodentechnolgie gestattet es ferner, planare Chipelektroden durch galvanisches Aufwachsen oder Grautonlithographie zu sphärischen Gebilden zu formen und sie auch in Verbundsystemen aus verschiedenen Chips räumlich oder planar zu kombinieren.

Die Anwendung von interdigitalen Arrays ist prinzipiell z. B. aus dem Artikel von M. Paeschke et al. "Properties of interdigital electrode arrays with different geometries" in Analytica Chimica Acta 305 (1995), S. 126 bis 136, für elektroanalytische Zwecke bekannt. Die erfindungsgemäße Mikroelektroden-Anordnung stellt eine Weiterentwicklung der dort dargestellten Grundkonzeption dar, bei der jedoch die gesamte Kammstruktur elektrisch leitfähig und damit als Elektrode ausgebildet ist.

Die dargestellten Mikroelektroden haben insbesondere bei der Messung des oben angegebenen MAP's hinsichtlich ihrer elektrischen Eigenschaften besondere Bedeutung. Die Phasengrenze zwischen Elektrode und Herzmuskelgewebe läßt sich elektrisch als Parallelschaltung eines Kondensators (der sogenannten "Helmholtz-Kapazität") und eines Widerstandes (des sogenannten "Faraday-Widerstandes") charakterisieren. Eine für die Messung von Aktionspotentialen ausreichend geringe Phasengrenz-Impedanz dieser Anordnung weisen die Elektroden dann auf, wenn sie geeignete Oberflächenbeschichtungen aufweisen. Letzteres kann durch eine fraktale oder elektroaktive Beschichtung erreicht werden. Von Vorteil ist ferner, daß aufgrund der Gatteranordnung die günstigen elektrischen Eigenschaften von Mikroelektroden wie hohe Stromdichte bei Stimulation und großer Frequenzbereich des Meßsignales bei Wahrnehmung, hervorgerufen durch die Dominanz des sphärischen Diffusionsanteiles gegenüber dem linearen Anteil, erhalten bleiben. Die Anwendung solcher Mikroelektroden-Gatter ist beispielsweise aus der US 4,969,468 A1 zur Messung an Zellgeweben oder Organen bekannt. Hierbei wird jedoch nur kapazitiv gemessen, da die eigentlichen Elektroden von einer dielektrischen Schicht abgedeckt sind.

In der vorliegenden Anwendung können zusammenfassend die Refraktärzeit und die Reizleitungsgeschwindigkeit im Herzmuskelgewebe erfindungsgemäß durch ein Mikroelektroden-Gatter erfaßt werden, wobei ein Vergleich des Produktes der beiden vorgenannten Werte mit jeweiligen Schwellwerten ausreicht, um entscheiden zu können, ob das Herz sich in einem tachykardiegefährdeten Zustand befindet. Ebenfalls anhand von Schwellwerten kann eine Einstufung des eventuell eingetretenen Tachykardie-Zustandes nach seiner Bedrohlichkeit für den Patienten vorgenommen und damit vom System eine geeignete Therapie ausgewählt werden. Mit Hilfe der erfindungsgemäßen Ultramikroelektroden können aufgrund besonders vorteilhafter Diffusionsverhältnisse Meßsignale mit wesentlich verbessertem Signal-Rausch-Verhältnis und erheblich erweitertem Frequenzbereich bei Wahrnehmung gewonnen werden. Darüber hinaus ist eine um Größenordnungen erhöhte Stromdichte bei Stimulationsprozessen möglich.

Abschließend wird darauf hingewiesen, daß in Fig. 3 die erfindungsgemäße Vorrichtung lediglich funktionell und blockdiagrammartig gezeigt ist. In der praktischen Umsetzung wird diese Vorrichtung auf Mikroprozessorbasis unter Steuerung durch funktionsgerechte Ablaufprogramme realisiert, wie dies aus der Herzschrittmacher-Technologie bekannt ist. Entsprechend kann die Steuereinrichtung 1 von außen telemetrisch umprogrammierbar sein, um die erfindungsgemäße Tachykardie-Früherkennungsvorrichtung sich ändernden kardiologischen Rahmenbedingungen anpassen zu können.

**Patentansprüche**

1. Implantierbare Vorrichtung zur Tachykardie-Früherkennung und

   - Unterdrückung beim Herzen mit
   - einer in Kontakt mit dem Herzmuskelgewebe stehenden Mikroelektroden-Anordnung (2) zur Erfassung von Reizleitungspotentialen im Herzmuskelgewebe,
   - einer mit der Mikroelektroden-Anordnung (2) in Verbindung stehenden Meßeinrichtung (12, 13, 14, 15, 16) zur Bestimmung der Reizleitungsgeschwindigkeit (v) im überwachten Herzmuskelbereich,
   - einer Stimulationsvorrichtung (19, 20, 21, 22) zur Erzeugung einer antitachykarden Stimulation bei Erfassung eines Tachykardie-gefährdeten Zustandes insbesondere über die Mikroelektroden-Anordnung (2), und
   - einer Steuereinrichtung (1) zur Steuerung der vorrichtungsinternen Meß- und Auswerteabläufe **gekennzeichnet durch**
   - eine mit der Mikroelektroden-Anordnung (2) in Verbindung stehende Meßeinrichtung (9, 10, 11) zur Bestimmung der Refraktärzeit ($t_R$) der Herzmuskelzellen im überwachten Herzbereich,
   - eine Recheneinrichtung (17) zur Bestimmung des Produktwertes aus Refraktärzeit ($t_R$) und Reizleitungsgeschwindigkeit (v),
   - einen Komparator (18) zum Vergleich des Produktwertes mit einem Tachykardie-Grenzwert (T), dessen Unterschreiten einen Tachykardiegefährdeten Zustand des Herzens signalisiert.

2. Vorrichtung nach Anspruch 1, **dadurch gekenn-**

**zeichnet, daß** die Mikroelektroden-Anordnung (2) ein planares Mikroelektroden-Gatter (23) mit kammartig ineinander greifenden, fingerartigen Elektrodenarmen (3) ist.

3. Vorrichtung nach Anspruch 1 oder 2, **dadurch gekennzeichnet, daß** die Mikroelektroden-Anordnung (2) auf einer in das Ventrikel oder Atrium einführbaren Elektrodensonde positioniert ist.

4. Vorrichtung nach einem der Ansprüche 1 bis 3, **dadurch gekennzeichnet, daß** mittels der Steuereinrichtung (1) über eine Beschaltungseinheit (4) paarweise Elektrodenarme (3) der Mikroelektroden-Anordnung (2) variabel zur Bestimmung von Refraktärzeit ($t_R$) und Reizleitungszeit (v) selektierbar sind.

5. Vorrichtung nach Anspruch 4, **dadurch gekennzeichnet, daß** die selektierten Elektrodenarme (3) mit zwei Eingangskanälen (5, 6) koppelbar sind, in denen jeweils Schwellwert-Komparatoren (9, 12) zur Signaldetektion eingeschaltet sind.

6. Vorrichtung nach Anspruch 5, **dadurch gekennzeichnet, daß** den Schwellwert-Komparatoren (9, 12) Zeitglieder (10, 14) zur Ermittlung der Refraktärzeit ($t_R$) und der Reizleitungszeit (v) zwischen den beiden selektierten Elektrodenarmen (3) zugeordnet sind.

7. Vorrichtung nach Anspruch 6, **gekennzeichnet durch** ein Rechenglied (16) zur Berechnung der Reizleitungsgeschwindigkeit (v) aus der Reizleitungszeit und dem über die Steuereinrichtung (1) vorgegebenen Abstand (a) der selektierten Elektrodenarme (3).

8. Vorrichtung nach einem der Ansprüche 1 bis 7, **dadurch gekennzeichnet, daß** der Steuereinrichtung (1) zwei Ausgangskanäle (21, 22) mit Ausgangsverstärkern (19, 20) als Stimulationseinrichtung nachgeschaltet sind.

9. Vorrichtung nach einem der Ansprüche 1 bis 8, **dadurch gekennzeichnet, daß** die Meß-, Rechen-, Komparator- und Steuereinrichtungen (1, 9, 10, 11, 12, 13, 14, 15, 16, 17, 18) auf Mikroprozessorbasis unter Steuerung durch funktionsgerechte Ablaufprogramme realisiert sind.

10. Vorrichtung nach einem der Ansprüche 1 bis 9, **dadurch gekennzeichnet, daß** die Vorrichtung von außen vorzugsweise telemetrisch umprogrammierbar ist.

11. Vorrichtung nach einem der Ansprüche 1 bis 10, **dadurch gekennzeichnet, daß** die Mikroelektroden-Anordnung (2) in Form eines Mikroelektroden-Gatters (23) mit fingerartig ineinandergreifenden Elektrodenarmen (3) eine isolierende Schicht (24) aufweist, deren Öffnungen (25) über den Elektrodenarmen (3) die Mikroelektroden bilden.

**Claims**

1. An implantable apparatus of the early diagnosis and suppression of tachycardia in the heart, comprising

   - a microelectrode array (2), which is in contact with the cardiac muscle tissue, for detecting stimulus conduction potentials in the cardiac muscle tissue,
   - a measuring device (12, 13, 14, 15, 16), which is in connection with the microelectrode array (2), for determining the stimulus conduction velocity (v) in the monitored cardiac muscle region,
   - a stimulation arrangement (19, 20, 21, 22) for generating an antitachy-cardia stimulation upon detection of a condition of risk of tachycardia in particular via the microelectrode array (2), and
   - a control unit (1) for controlling the measuring and evaluation processes within the apparatus **characterized by**
   - a measuring device (9, 10, 11), which is in connection with the microelectrode array (2), for determining the refractory time ($t_R$) of the cardiac muscle cells in the monitored cardiac muscle region,
   - a computing device (17) for determining the product value of the refractory time ($t_R$) and the stimulus conduction velocity (v),
   - a comparator (18) for comparing the product value with a tachycardia threshold (T), any falling short of the tachycardia threshold (T) signaling a condition of the heart in risk of tachycardia.

2. An apparatus according to claim 1, **characterized in that** the microelectrode array (2) is a planar microelectrode gate (23) with meshing, finger-type electrode arms (3).

3. An apparatus according to claim 1 or 2, **characterized in that** the microelectrode array (2) is positioned on an electrode probe to be inserted into the ventriculus or atrium.

4. An apparatus according to one of claims 1 to 3, **characterized in that** for determination of the refractory time ($t_R$) and the stimulus conduction time (v), pairs of electrode arms (3) of the microelectrode array (2) are variably selectable by means of the control unit (1) via a connecting unit (4).

5. An apparatus according 4, **characterized in that** the selected electrode arms (3) can be coupled with two input channels (5, 6), in each of which threshold comparators (9, 12) are interconnected for signal detection.

6. An apparatus according to claim 5, **characterized in that** the threshold comparators (9, 12) are provided with time function elements (10, 14) for determination of the refractory time ($t_R$) and the stimulus conduction time (v) between the two selected electrode arms (3).

7. An apparatus according to claim 6, **characterized in that** a computing element (16) for calculating the stimulus conduction velocity (v) from the stimulus conduction time and the distance (a), preset by the control unit (1), of the selected electrode arms (3).

8. An apparatus according to one of claims 1 to 7, **characterized in that** the control unit (1) is followed by two output channels (21, 22) comprising output amplifiers (19, 20) as a stimulation device.

9. An apparatus according to one of claims 1 to 8, **characterized in that** the measuring, computing, comparator and control devices (1, 9, 10, 11, 12, 13, 14, 15, 16, 17, 18) are put into practice on a microprocessor basis, controlled by functional, operational programs.

10. An apparatus according to one of claims 1 to 9, **characterized in that** the apparatus is reprogrammable preferably telemetrically from outside.

11. An apparatus according to one of claims 1 to 10, **characterized in that** the microelectrode array (2) in the form of a microelectrode gate (23) which has electrode arms (3) meshing in the way of fingers comprises an insulating layer (24), the openings (25) of which located over the electrode arms (3) constitute the microelectrodes.

**Revendications**

1. Dispositif implantable pour le coeur destiné à la détection précoce et à la suppression de la tachycardie

   • avec un arrangement de microélectrodes (2) qui sont en contact avec le tissu du muscle cardiaque et qui servent à enregistrer les potentiels de transmission de l'excitation dans le muscle cardiaque,
   • avec une installation de mesure (12, 13, 14, 15, 16) qui est en liaison avec l'arrangement de microélectrodes (2) et qui est destinée à déterminer la vitesse (v) de transmission de l'excitation dans la zone surveillée du muscle cardiaque,
   • avec un dispositif de stimulation (19, 20, 21, 22) destiné à produire une stimulation antitachycardique lors de la détection d'un état de risque tachycardique en particulier au moyen de l'arrangement de microélectrodes (2) et
   • avec une installation de commande (1) destinée à commander les processus de mesure et d'interprétation internes aux dispositifs,

   **caractérisé par**

   • une installation de mesure (9, 10, 11) qui est en liaison avec l'arrangement de microélectrodes (2) et qui est destinée à déterminer le temps de réfraction ($t_R$) des cellules du muscle cardiaque dans la zone cardiaque surveillée,
   • une installation de calcul (17) destinée à déterminer la valeur du produit du temps de réfraction ($t_R$) et de la vitesse (v) de transmission de l'excitation,
   • un comparateur (18) destiné à comparer la valeur du produit avec une valeur limite de tachycardie (T) dont le dépassement de la limite inférieure indique un état du coeur à risque tachycardique.

2. Dispositif selon la revendication 1 **caractérisé en ce que** l'arrangement de microélectrodes (2) est une grille de microélectrodes (23) planaire avec des languettes d'électrodes (3) en forme de doigts qui s'interpénètrent à la manière d'un peigne.

3. Dispositif selon la revendication 1 ou 2 **caractérisé en ce que** l'arrangement de microélectrodes (2) est placé sur une sonde d'électrodes qu'on peut introduire dans le ventricule ou dans l'oreillette.

4. Dispositif selon l'une des revendications 1 à 3 **caractérisé en ce que**, au moyen de l'installation de commande (1) par l'intermédiaire d'une unité de connexion (4), des languettes d'électrodes (3) de l'arrangement de microélectrodes (2) peuvent être sélectionnées par paires de façon variable pour déterminer le temps de réfraction ($t_R$) et la vitesse (v) de transmission de l'excitation.

5. Dispositif selon la revendication 4 **caractérisé en ce que** les languettes d'électrodes (3) sélectionnées peuvent être couplées avec deux canaux d'entrée (5, 6) dans lesquels sont à chaque fois connectés des comparateurs de valeur seuil (9, 12) destinés à détecter des signaux.

6. Dispositif selon la revendication 5 **caractérisé en**

**ce que** des unités temporelles (10, 14), destinées à établir le temps de réfraction ($t_R$) et le temps (v) de transmission de l'excitation entre les deux languettes d'électrodes (3) sélectionnées, sont associées aux comparateurs de valeur seuil (9, 12).

7. Dispositif selon la revendication 6 **caractérisé par** une unité de calcul (16) destinée à calculer la vitesse (v) de transmission de l'excitation à partir du temps de transmission de l'excitation et de la distance (a) - prédéfinie par l'installation de commande (1) - des languettes d'électrodes (3) sélectionnées.

8. Dispositif selon l'une des revendications 1 à 7 **caractérisé en ce que** deux canaux de sortie (21, 22) avec des amplificateurs de sortie (19, 20) en tant qu'installations de stimulation sont connectés en aval de l'installation de commande (1).

9. Dispositif selon l'une des revendications 1 à 8 **caractérisé en ce que** les installations (1, 9, 10, 11, 12, 13, 14, 15, 16, 17, 18) de mesure, de calcul, de comparaison et de commande sont réalisées à partir de microprocesseurs commandés par des programmes de déroulement adaptés à la fonction.

10. Dispositif selon l'une des revendications 1 à 9 **caractérisé en ce que** le dispositif peut être programmé de l'extérieur, par exemple par télémesure.

11. Dispositif selon l'une des revendications 1 à 10 **caractérisé en ce que** l'arrangement de microélectrodes (2), sous forme d'une grille de microélectrodes (23) avec des languettes d'électrodes (3) qui s'interpénètrent à la manière de doigts, présente une couche isolante (24) dont les ouvertures (25) sur les languettes d'électrodes (3) forment les microélectrodes.

FIG.1

FIG.2

FIG.5

FIG.4

FIG. 3